# EUROPEAN PATENT APPLICATION

(11) **EP 3 494 970 A2**
(43) Date of publication of application: **12.06.2019**
(21) Application number: 18020630.2
(22) Date of filing: 07.12.2018
(51) Int. Cl.: A61K 31/4985, A61K 9/00, A61K 47/26, A61K 47/38

(54) **HARDLY SOLUBLE THERAPEUTIC AGENTS BELONGING TO BCS CLASS II OR IV SUSPENDED IN THE LIQUID FORMULATION AND/OR IN THE FINAL NANOFIBROUS STRUCTURE**

(30) Priority: 05.12.2017 CZ 20170781
(71) Applicant: Zentiva K.S., 102 37 Praha 10 (CZ)
(72) Inventor: Kluk, Anna, 10-691 Olsztyn (PL); Gajdosova, Michaela, 98401 Lucenec (SK); Hanzlik, Pavel, 190 00 Praha 9 (CZ); Sedlak, Pavel, 149 00 Praha 4 (CZ)
(74) Representative: Jirotkova, Ivana

(57) **Abstract**

The present solution relates to the composition of the stable suspension used for electrospinning process in order to prepare orodispersible films, which comprises hardly soluble therapeutic agents, suspended in the viscous solution of one or more suitable pharmaceutical excipients. In the preferred embodiment the amount of API is 1.0 - 30.0% by weight of the total suspension.

## Description

### Field of the Invention

The present invention relates to the composition of the liquid pharmaceutical formulation used for electrospinning process as well as the composition of obtained orodispersable films (ODFs), comprising at least one of the hardly soluble therapeutic agents, belonging to the BCS class II and IV, where at least one pharmaceutically active substance is not dissolved, but suspended. The invention further relates to preparation method of the homogenous suspension or nanosuspension, which remains stable throughout the whole electrospinning process, even without stirring, providing enhanced process efficiency and high API content in the received films.

### Background Art

Orodispersible films (ODFs) have recently gained much attention, as it is a very beneficial formulation for pediatric and geriatric patients, where the difficulty of swallowing of the standard solid drug forms needs to be eliminated. Orodispersible films either disintegrate quickly in the oral cavity and can be swallowed easily or stay in the particular place in the mouth due to their mucoadhesive properties. Thus ODFs are used as rapid release products and as buccoadhesive or swallowed controlled drug release systems.

Generally, oral strips are manufactured by casting method - preparation of solution or suspension, which is left for solvent evaporation. In this method melt polymer can also be used. Unfortunately this production method is considered as problematic, since it is difficult to achieve homogenous assay and thickness of the films, especially in a bigger scale. Other techniques used for ODFs manufacturing are hot melt extrusion and ink jet printing on placebo films (J. Control Release 206 (2015) 1-19). The newest method of ODF production is electrospinning - a process, where polymer nanofibers (diameter from few nanometers to several micrometers) can be produced using an electrostatically driven jet of polymer solution or polymer melt. The advantages of electrospinning method for ODFs production have been described in detail in a Zentiva's previous patent application (EP2813212A1). Due to the porous structure of polymeric nanofibers, obtained ODFs, in comparison with products obtained by casting method, demonstrate favorable properties, including homogeneity of thickness and assay, faster disintegration and immediate dissolution.

The use of electrostatic forces to form ultrafine fibers was first patented by Formhals in 1934 (US patent 1975504, 1934). Since then, this technique had been known as electrostatic spinning and had gained much interest on different applications, including filtration (J. Membr. Sci. 281 (2006) 581-586, J. Membr. Sci. 315 (2008) 11-19, J. Adv. Mater. 34 (2002) 44-55), cosmetic mask (CN101390814-A, US2009031691-A1), military protective clothing (US patent KR2010048661-A, J. Appl. Polym. Sci. 102 (2006) 3430-3437, J. Appl. Polym. Sci. 125 (2012) 4135-4141), nano-sensor (IEEE Sens. J. 8 (2008) 951-953, Biomacromolecules 9 (2008) 2087-2090), energy-related applications (J. Power Sources 196 (2011) 4886-4904), wound dressings (Polym. Adv. Technol. 21 (2010) 77-95, J. Biomed. Mater. Res. B. 67B (2003) 675-679, J. Mater. Chem. B. 1 (2013) 4531-4541), drug delivery (J. Nanomater. (2013) 1-22, Biomaterials 29 (2008) 1989-2006), enzyme immobilization (Acta Biomater. 4 (2008) 1770-1777, Biomaterials 29 (2008) 1118-1126), and tissue engineering scaffolds (Tissue Eng. 12 (2006) 1197-1211, Adv. Drug Deliv. Rev. 61 (2009) 1033-1042).

Among the various potential applications, drug delivery is one of the most promising uses. From the first study on the application of electrospun nano-fibers for the sustained release of tetracycline hydrochloride by Kenawy et al. (J. Control. Release 81 (2002) 57-64), electrospun nanofibers have been successfully used to achieve different drug release profiles (Adv. Polym. Sci. 246 (2012) 241-262, J. Mater. Sci. - Mater. M. 16 (2005) 933-946). Among them immediate release formulations are most popular, as electrospinning provides the opportunity to improve dissolution and bioavailability, especially for poorly-soluble drugs. During the process amorphous materials with improved water solubility of the APIs are prepared (Eur. J. Pharm. Sci., (2013) 49 (4) 595-602). Moreover, the film can disintegrate quickly upon contact with water, due to a very large surface area of nanofibers and water soluble excipients used for their production. Thanks to these features ODFs can become a beneficial oral dosage forms, characterized by immediate release, even for hardly-soluble drugs under physiological conditions.

One of the most important requirements to use electrospinning for oral strips production is to prepare the final formulation in the form of solution. This form is easy to handle and can provide desirable homogeneity of the film and a proper drug content uniformity in the final strips. When using water-soluble polymers water can be applied as solvent in the electrospinning process, however, it is not sufficient to dissolve poorly soluble drugs and requires the addition of organic solvents. Different solvents from this group (ethanol, methanol, dichloromethane, chloroform, trifluoroethanol, tetrahydrofuran) can be applied in the process of electrostatic spinning, but this can be considered a disadvantage for the oral formulation due to the safety issues (Eur. J. Pharm. Sci., (2013) 49 (4) 595-602). The problem could be solved by using suspension form instead of solution (API particles suspended in the viscous medium). However, as the mixing of the liquid formulation during the electrospinning process still remains a challenge, using of standard suspension is also problematic, because without continuous mixing sedimentation may occur, which may cause inhomogeneous drug distribution within the film layer.

### Disclosure of Invention

The present invention relates to the composition of the stable suspension used for electrospinning process in order to prepare orodispersible films, which comprises hardly soluble therapeutic agents, suspended in the viscous solution of one or more suitable pharmaceutical excipients. In the preferred embodiment the amount of API is 1.0 - 30.0% by weight of the total suspension.

In another preferred embodiment the claimed composition comprises hardly soluble APIs, with appropriate particle size distribution, obtained by any technique well known in the art, wherein 90% of particles or more is bigger than 100 nm and smaller than 50 µm, preferably equal or bigger than 500 nm and equal or smaller than 10 µm.

In another preferred embodiment the claimed composition of suspension apart from API, consists of the surfactants, water soluble polymers, sweeteners and optionally taste masking agents, aromas, flavours, which are completely dissolved or dispersed in the formulation.

The invention provides also the proper selection and proportion between surfactants and solvents, for obtaining viscous solution of suitable excipients, which is a proper matrix for stable viscous suspension of homogenously dispersed API. Obtained suspension is stable, even without mixing and can be electrospun into homogenous layer, characterized by favorable properties, including proper content uniformity and acceptable purity without residual solvents.

The present invention also relates to the method of preparation of stable suspension, used for electrospinning process, comprising completely dissolved excipients mentioned above and homogenously suspended API. The method is divided into two steps:
1. API milling with a part of excipients
2. Preparation of viscous medium from the rest of excipients,
3. Mixing of both phases, addition of flavor, optionally colorant.
Or:
1. Preparation of viscous solution from all of excipients,
2. Homogenization with micronized API,
3. Addition of flavor, optionally colorant.

In another preferred embodiment the pharmaceutical composition is in the form of orodispersable strips, containing suspended API and characterized by sufficient purity and fast disintegration time.

The invention provides a pharmaceutical composition of ODFs, obtained by electrospinning of the claimed suspension. It comprises at least one of the poorly water-soluble active ingredients, suspended in the nanofibrous film structure, consisting of water soluble polymers and one or more suitable pharmaceutical excipients.

### Detailed description of Invention

The objective of the present invention is to provide a novel pharmaceutical composition of orodispersable films, prepared by electrospinning method, comprising at least one of hardly-soluble active pharmaceutical agents, suspended in the polymeric film structure. Obtained strips are characterized with favorable properties, including faster disintegration, proper drug content uniformity and suitable purity profile.

Oral strips are prepared from a stable, homogenous suspension of at least one hardly soluble API, suspended in the viscous solution of selected excipients. Authors of the present invention found that suitable API PSD (nanomilled or micronized) provides sufficient stability and homogeneity of obtained suspension, which can be effectively electrospun (even without continuous mixing) into homogenous layer of immediate disintegration, desirable content uniformity and low content of residual solvents.

The present invention relates to the composition of the stable suspension used for electrospinning process in order to prepare orodispersible films, which comprises hardly soluble therapeutic agents, together with one or more suitable pharmaceutical excipients.

In the preferred embodiment the suspension prepared for orodispersible films using the method of electrospinning, contains 1-30% of a hardly soluble active ingredient, and 2 to 30% of water soluble long-chained film forming polymers. This preferred suspension is characterized by that the sedimentation, evaluated based on assay difference between bottom and top layers of the 30-cm column of unmixed suspension, is no greater than 2.5%.after 6 or preferable 8 hours without any agitation.

The term "hardly soluble" means the drug being classified in the BCS II or IV. The drugs which are not "highly soluble" are mostly within this classification. The "highly soluble" drug is defined by that the highest therapeutic dose is dissolved in a volume of 250 ml of a solution with a pH in the range of 1-7.5 and at 37 ° C . Preferable the "hardly soluble drugs" provide lower solubility than 0.16 mg/ml.

In the preferred embodiment the amount of API is 1.0 - 30.0% by weight of the total suspension. The API used in the composition is either micronized, where 90% or more of the particles have a size of 1-30 µm or nanomilled, where 90% or more of the particles have a size of 100-1000 nm. The claimed particle size distribution of API provides a sufficient stability of prepared suspension within the time needed for electrospinning time performance (no less than 8h).

The claimed composition of suspension consists of:
- 2-30% of water soluble long-chained polymers
- up to 2.0% of surfactants,
- up to 0.5% of sweeteners,
- optionally up to 5.0% of aromas and flavours,
- optionally up to 1.0% of the pharmaceutically accepted colorants,
- and 10-90% of solvents.

The main components of the above formulation are long-chained water soluble polymers. The polymer solution has to be viscous enough to prevent a sedimentation but on the other to allow a continuous dosing of the suspension to the equipment to provide homogenous units of the oral dispersible films. The films formed by the polymer should provide patients with the best compliance. Examples of the polymers are artificial and natural polymers such as, but not limited to cellulose and its derivatives (preferably methylcellulose, hydroxyethylcellulose, hydroxypropylcellulose, hydroxypropylmethylcellulose, sodium carmellose), polyoxyethylene glycols and their derivatives and copolymers, polysaccharides (preferably pullulan), polyacrilic acid, its derivatives and copolymers, methacrylic acid derivatives, xanthan gum, alginates, carrageenan, pectins, Gantrez, maltodextrins or, preferably, their mixtures.

Preferably viscosity of the suspension is lower than 500 mPas,, for example in some embodiment the viscosity was adjusted between 300 to 400 mPas.

In the most preferred embodiment of the present invention the mixture of hydroxylpropylmethyl cellulose and polyoxyethylene glycol was used. This selection of polymeric components led to achieve the high productivity of electrospinning process and obtain adequate mechanical properties of the final ODFs (elasticity, homogeneity, appearance of the films, quality of nanofibers), even with high API content in the film. It was observed that nanofibrous films based either on HPMC alone or the mixture of HPMC and PVP/PVA are brittle and fragile, while using PVP or PVA alone or in the mixture with PEG resulted in the stickiness and sensitivity to touch of the final ODFs.

In order to achieve stable solution, polymeric ingredients (HPMC, PEG) were used in the particular ratios (preferably 9.5:0.5, 9:1, 8:2, 7:3 and 6:4, respectively). These proportions between polymers led to obtain clear solutions, characterized by an adequate viscosity and physical stability for at least 3 days (no precipitation, viscosity change, liquid phase separation, conductivity, density). Moreover, described properties (viscosity, density) guaranteed homogeneous distribution of API in the suspension and its sufficient stability during electrospinning process.

In order to obtain an attractive taste of the final ODFs, the formulation also comprises at least one sweetener (preferably thaumatin, sucralose, aspartame, acesulfame potassium) at concentration up to 0.5% of total weight of suspension, preferably up to 0.2%, optionally at least one flavour (preferably mint, orange, lemon, strawberry, raspberry, cherry, ginger) at concentration up to 5%, as well as optionally at least one of the pharmaceutically accepted colorants and dyes, at concentrations up to 2% of total weight of solution.

The invention provides also the proper selection and proportion between surfactants and solvents, which result in obtaining of a clear or colloidal solution of chosen excipients and homogenous dispersion of hardly soluble APIs in the formulation. In the present invention a mixture of water and isopropanol was used, mixed in different ratios (preferably 1:1, 1:2, 1:3, 1:4, 2:3, 2:5, 3:5, 4:5, respectively). Addition of different surfactants, preferably sodium lauryl sulfate or Tweens in different concentrations up to 2.0% helped with preparation of suspension, especially in the first step of nanomilling, if it has been applied. The claimed composition allowed for preparation of stable viscous suspensions, containing completely dissolved excipients and undissolved API particles, which have been electrospun into homogenous layers, characterized by desirable content uniformity and low content of residual solvents. The proper ratios between solvents provided sufficient stability of hardly soluble API in the formulation and prevented API against dissolving and potential recrystallization within a time.

The present invention also relates to the method of preparation of stable suspension, comprising suspended and homogenously dispersed API and dissolved excipients mentioned above, used for electrospinning process. Stability of the solution depended not only on the appropriate balance between ingredients and solvents, but also on the suitable API PSD, which has been obtained thanks to preliminary micronization (or nanomilling) step.

Examples of useful drugs, belonging to II or IV BCS class, include anti-emetics, antinauseants, cardiovascular agents, antianginal drugs, anti-arrhythmics, anti-coagulants, antithrombotic drugs, coronary dilators, anti-hypertensive drugs, central nervous system stimulants, dopamine receptor agonists, anti-anxiety agents, cholinesterase inhibitors, anesthetics, anti-convulsants, hypnotics, anti-depressants, anti-parkinsonian agents, psychotherapeutic agents, anti-diabetic agents, anti-cholesterolemics, premature ejaculation management agents, drugs used to treat erectile dysfunction, parasympatholytics, parasympathomimetics, motion sickness management agents, anti- diarrhea preparations, antidotes, anti-histamines, anti-inflammatory agents, anti-lipid agents, analgesics, antiasthmatics, anti-stroke agents, anti-thyroid preparations, antitumor drugs, anti-viral agents, acne drugs, alkaloids, amino acid preparations, anti- tussives, anti-uricemic drugs, anabolic preparations, systemic and non- systemic anti-infective agents, anti-neoplastics, anti-rheumatic agents, appetite stimulants, biological response modifiers, blood modifiers, bone metabolism regulators, contraceptives, decongestants, endometriosis management agents, enzymes, fertility agents, gastrointestinal agents, hormones, hypercalcemia and hypocalcemia management agents, immunomodulators, immunosuppressives, migraine preparations, muscle relaxants, obesity management agents, osteoporosis preparations, oxytocics, prostaglandins, respiratory agents, sedatives, smoking cessation aids, sympatholytics, tremor preparations, urinary tract agents, vasodilators, laxatives, antacids, anti-pyretics, appetite suppressants, expectorants, anti-ulcer agents, anti-inflammatory substances, cerebral dilators, peripheral vasodilators, psycho-tropics, stimulants, vasoconstrictors, migraine treatments, antibiotics, tranquilizers, anti-psychotics, neuromuscular drugs, hyper-and hypo-glycemic agents, thyroid and anti-thyroid preparations, diuretics, ariti-spasmodics, erythropoietic drugs, cough suppressants, mucolytics, DNA and genetic modifying drugs, proteins, peptides and combinations thereof.

Non-limiting examples of active pharmaceutical agents used according to the present invention are atorvastatin, bisacodyl, carbegoline, candesartan cilexetil, carvedilol, chlorpromazine, clozapine, dexamethasone, diazepam, diclofenac, ebastine, ezitimib, famotidine, furosemid, glimepiride, glipizide, glibenclamide, haloperidol, hydrochlorothiazide, ketoprofen, lamotrigine, loratadine, lorazepam, lovastatin, meloxicam, metoclopramide, mosapride, nifedipine, nilvadipine, olanzapine, pioglitazone, risperidone, rofecoxib, tadalafil, simvastatin, telmisartan, warfarin and combinations thereof.

The claimed method of suspension preparation consists of the following points:
1. Micronization (or nanomilling) of API with a part of excipients dissolved in the mixture of solvents (preferably with water, surfactant and polymer),
2. Preparation of viscous solution from the rest of excipients in the mixture of solvents, preferably water and organic miscible solvent,
3. Mixing of both phases, optionally addition of flavour, colorant,
4. Electro spinning.

Or:
1. Preparation of viscous solution from all of excipients (except of flavour and colorant) in the mixture of solvents, preferably water and organic miscible solvent,
2. Homogenization with micronized API,
3. optionally addition of flavour, colorant,
4. Electro spinning.

In another preferred embodiment the claimed composition of suspension results in the following composition of oral strip:
- 1 - 30% of micronized or nanomilled API belonging to II or IV BCS class,
- 2 - 30% of water soluble long-chained, artificial and natural polymers such as, but not limited to cellulose and its derivatives (preferably methylcellulose, hydroxyethylcellulose, hydroxypropylcellulose, hydroxypropylmethylcellulose, sodium carmellose), polyoxyethylene glycols and their derivatives and copolymers, polysaccharides (preferably pullulan), polyacrilic acid, its derivatives and copolymers, methacrylic acid derivatives, xanthan gum, alginates, carrageenan, pectins, Gantrez, maltodextrins or their mixtures,
- up to 2.0% of surfactants, preferably from the group of non-ionic, anionic, cationic, amphiphilic surfactants
- up to 0.5% of sweeteners (preferably thaumatin, sucralose, aspartame, acesulfame potassium, stevia) and their mixtures
- optionally up to 5.0% of aromas and flavours (preferably mint, orange, lemon, orange, lemon, strawberry, raspberry, cherry, ginger) and their mixtures
- optionally up to 1.0% of the pharmaceutically accepted colorants, which can be used in drug products (preferably E100, E101, E102, E104, E110, E120, E122, E123, E124, E131, E132, E133, E141, E142,E160a-e, E162)
- 10-90% of solvents, preferably water, aqueous buffers and water miscible organic solvents, such as but not limited to isopropanol, ethanol, methanol, acetonitrile, acetone, ethylene and propylene glycols, THF, DMF, DMAC, PEGs and their mixtures.

In presented invention ODFs comprise at least one pharmaceutically active substance, deposited in and/or on at least one active layer of nanofibers of biologically compatible material. Preferably, ODFs consist of more than one layer, of which at least one is prepared by electrospinning method and can be combined with other layers produced by any other method. In another preferred embodiments ODFs consist of more than one layer, where each layer can have the same or different composition. Preferably external polymeric layers have particular composition (i. e. polymers and taste masking excipients), while internal layer(s) consist(s) of different ingredients and contain only API in the matrix ("sandwich model"). In another preferred embodiment ODFs are prepared by electrospinning method in the way providing that the film is free of residual solvents.

The films of the present invention may be mucoadhesive, whose advantage resides in their ability to bypass the gastrointestinal tract, and barriers in the gastrointestinal tract to drug absorption such as first pass metabolism and decomposition of the active ingredient in the stomach.

Alternatively the films of the present invention may be non-mucoadhesive, which mimics the pharmacokinetic profile conventional immediate release solid oral dosage forms and are bioequivalent to and interchangeable with existing orally administered drug products. The films of the present invention must be formed into a sheet or film prior to drying. After the desired components are combined to form a multi-component matrix, including the polymer, water, and active or other components as desired, the combination is formed into a film, by electrospinning method.

In another embodiment solutions or dispersions are electrospun on supportive material such as, but not limited to polypropylene textile, baking paper, siliconized paper, edible paper, aluminium foil. The carrier can be continuously removed during the electrospinning process, or afterwards, before cutting of the film into pieces (PP textile, baking paper, aluminium foil). Preferably, it can be an integral part of the final product (edible paper).

The API is e.g. apixaban, bisacodyl, dexamethasone, dicycloverine, dimenhydrinate, loperamide, loratadine, lornoxicam, meloxicam, olanzapine piroxicam, prochlorperazine, risperidone, suvorexant, vardenafil, vinpocetine, zafirlukast, zopiclone.. The polymers used for these APIs are cellulose derivatives, preferably hydroxypropyl cellulose.

The invention can be used for example for tadalafil in which the polymer concentration can be from 7 to 14% w/w and 5 to 8% of suspended active ingredient. The suspension viscosity can be of the value 300 to 400 mPas. The dissolved polymer can be selected as a combination HPMC and PEG in the ration 8 : 2 to 6 : 4.

The parameters of electrospinning process depend on the specific machine to be used. For example they can be adjusted: electrode distance 130 to 190 mm, rewinding speed 5 to 20 mm/ min; HV CE (high voltage collecting electrode) supply voltage -20 to -5 kV; HV SE (high voltage spinning electrode) supply voltage 30 to 70 kV and the air humidity 0 to 50% RH.

### Examples

### Example 1

| **Ingredient** | **Composition** | | |
|---|---|---|---|
| | **% in the liquid** | **% in ODF** | **mg/ODF 20x25mm (5 cm²)** |
| Phase I (nanomilling) | | | |
| Tadalafil | 6.75 | 40.13 | 5.00 |
| HPMC | 2.25 | 13.38 | 1.66 |
| Sodium lauryl sulfate | 0.068 | 0.40 | 0.05 |
| Water | 45.00 | - | - |

| Phase II (viscous solution) | | | |
|---|---|---|---|
| HPMC | 4.75 | 28.24 | 3.52 |
| PEG 100 000 | 3.00 | 17.85 | 2.22 |
| Isopropanol | 45.00 | - | - |
| Total: | 106.818 | 100.00 | 12.45 |
| Solids: | 16.818 | | |

### Suspension preparation:

6.75 g of tadalafil was dispersed in the aqueous solution of 2.25 g of HPMC and 0.068 g of SLS, then nanomilled till targeted API PSD d(0.9) < 500 nm in the laboratory ball mill with the parameters presented below.

| ***Nanomilling*** | Balls volume (ml) | Balls size (µm) | Milling speed (rpm) | Milling time (min) |
|---|---|---|---|---|
| | 14 | 300 | 7000 | 30 |

The remaining polymers (4.75 g of HPMC and 3.00 g of PEG 100 000) were dissolved in isopropanol and after a clean solution was obtained, it was mixed with prepared API nanosuspension. The obtained liquid formulation, containing 6.3% of tadalafil, was left under continuous stirring for about 30 min and then subjected to electro spinning. An one-layer film of the average weight 2.5 mg/ cm² was collected on the polypropylene textile. After separation from the carrier, the film was cut into 25 x 20 mm strips, containing 5.0 mg of tadalafil per strip.

One layer weight 2.45-2.55 mg/cm²
Tadalafil dose: 1.003 mg /cm²

### Process parameters:

Equipment: laboratory scale Nanospider - needleless electrospinning
Electrode distance [mm] 140
Rotation/wire speed [mm] 15
Rewinding speed [mm/min] 5
EMW speed [mm /sec] 300
Air Input- flow [m3/h] 170.0
Air Output- flow [m3/h] 58.0
High Voltage setup
   HV CE Supply current [mA] 0.25
   HV CE Supply voltage [kV] -10.0
   HV SESupply current [mA] 0.25
   HV SE Supply voltage [kV] 50.0

### Example 2

| **Ingredient** | **Composition** | | |
|---|---|---|---|
| | **% in the liquid** | **% in ODF** | **mg/ODF 20x22.5 mm (4.45 cm²)** |
| Phase I (nanomilling) | | | |
| Tadalafil | 6.75 | 40.13 | 5.00 |
| HPMC | 2.25 | 13.38 | 1.66 |
| Sodium lauryl sulfate | 0.068 | 0.40 | 0.05 |
| Water | 45.00 | - | - |

| Phase II (viscous solution) | | | |
|---|---|---|---|
| HPMC | 4.75 | 28.24 | 3.52 |
| PEG 100 000 | 3.00 | 17.85 | 2.22 |
| Isopropanol | 45.00 | - | - |
| Total: | 106.818 | 100.00 | 12.45 |
| Solids: | 16.818 | | |

### Suspension preparation:

6.75 g of tadalafil was dispersed in the aqueous solution of 2.25 g of HPMC and 0.068 g of SLS, then nanomilled till targeted API PSD d(0.9) < 1000 nm in the laboratory ball mill with the parameters presented below.

| ***Nanomilling*** | Balls volume (ml) | Balls size (µm) | Milling speed (rpm) | Milling time (min) |
|---|---|---|---|---|
| | 14 | 300 | 4000 | 20 |

The remaining polymers (4.75 g of HPMC and 3.00 g of PEG 100 000) were dissolved in isopropanol and after a clean solution was obtained, it was mixed with prepared API nanosuspension. The obtained liquid formulation, containing 6.3% of tadalafil, was left under continuous stirring for about 30 min and then subjected to electro spinning. An one-layer film of the average weight 2.8 mg/ cm² was collected on the polypropylene textile. After separation from the carrier, the film was cut into 25 x 20 mm strips, containing 5.0 mg of tadalafil per strip.

One layer weight 2.68-2.85 mg/cm²
Tadalafil dose: 1.12 mg /cm²

### Process parameters:

Equipment: laboratory scale Nanospider - needleless electrospinning
Electrode distance [mm] 170
Rotation/wire speed [mm] 15
Rewinding speed [mm/min] 5
EMW speed [mm /sec] 300
Air Input- flow [m3/h] 170.0
Air Output- flow [m3/h] 127.0
High Voltage setup
   HV CE Supply current [mA] 0.18
   HV CE Supply voltage [kV] -10.0
   HV SE Supply current [mA] 0.17
   HV SE Supply voltage [kV] 50.0

### Example 3

| **Ingredient** | **Composition** | | |
|---|---|---|---|
| | **% in the suspension** | **% in ODF** | **mg/ODF 20x27 mm (5.40 cm²)** |
| Tadalafil micronized (d(0.9) < 8 um) | 7.500 | 42.03 | 10.00 |
| HPMC | 7.000 | 39.23 | 9.33 |
| Sodium lauryl sulfate | 0.075 | 0.42 | 0.10 |
| PEG 100 000 | 3.000 | 16.81 | 4.00 |
| sucralose | 0.020 | 0.11 | 0.03 |
| Spearmint aroma | 0.250 | 1.40 | 0.33 |
| Isopropanol | 45.000 | - | - |
| Water | 45.000 | - | - |
| Total: | 107.845 | 100.00 | 23.79 |
| Solids: | 17.845 | | |

### Suspension preparation:

0.075 g of SLS has been dissolved in the mixture of isopropanol and water (9.00 g and 11.25 g, respectively), then 7.50 g of tadalafil was dispersed in the obtained solution. HPMC (7.00 g) PEG 100 000 (3.00 g) and sucralose (0.02 g) were dissolved also in the mixture of both diluents (33.75 g of isopropanol and 31.5 g of water). Then API dispersion was added to the polymer solution, rinsing the beaker with the rest of solvents. Both phases were mixed together until homogenous suspension was achieved. Spearmint aroma (0.25 g) was added at the end, and the whole dispersion was mixed continously until subjected to electrospinning. An one-layer film of the average weight 4.40 mg/ cm² was collected on the polypropylene textile. After separation from the carrier, the film was cut into 20 x 27 mm strips, containing 10.0 mg of tadalafil per strip.

One layer weight 4.30 - 4.50 mg/cm²
Tadalafil dose: 1.85 mg /cm²

### Process parameters:

Equipment: laboratory scale Nanospider - needleless electrospinning
Electrode distance [mm] 170
Rotation/wire speed [mm] 15
Rewinding speed [mm/min] 5
EMW speed [mm /sec] 300
Air Input- flow [m3/h] 170.0
Air Output- flow [m3/h] 60
High Voltage setup
   HV CE Supply current [mA] 0.17
   HV CE Supply voltage [kV] -10.0
   HV SE Supply current [mA] 0.17
   HV SESupply voltage [kV] 50.0

### Example 4

| **Ingredient** | **Composition** | | |
|---|---|---|---|
| | **% in the suspension** | **% in ODF** | **mg/ODF strip (5.6 cm²)** |
| Tadalafil micronized (d(0.9) < 9 um) | 9.000 | 47.00 | 20.00 |
| HPMC | 7.000 | 36.55 | 15.55 |
| Sodium lauryl sulfate | 0.075 | 0.39 | 0.17 |
| PEG 100 000 | 3.000 | 15.66 | 6.66 |
| Sucralose | 0.075 | 0.39 | 0.17 |
| Isopropanol | 45.000 | - | - |
| Water | 45.000 | - | - |
| Total: | 109.90 | 100.00 | 42.55 |
| Solids: | 19.15 | | |

### Suspension preparation:

0.075 g of SLS has been dissolved in the mixture of isopropanol and water (9.00 g and 11.25 g, respectively), then 9.00 g of tadalafil was dispersed in the obtained solution. HPMC (7.00 g) PEG 100 000 (3.00 g) and sucralose (0.075 g) were dissolved also in the mixture of both diluents (33.75 g of isopropanol and 31.5 g of water). Then API dispersion was added to the polymer solution, rinsing the beaker with the rest of solvents. Both phases were mixed together until homogenous suspension was achieved. The whole dispersion was mixed continuously until subjected to electro spinning. An one-layer film of the average weight 7.30 mg/ cm² was collected on the polypropylene textile. After separation from the carrier, the film was cut into 25 x 22 mm strips, containing 20.0 mg of tadalafil per strip.

One layer weight 7.25 - 7.35 mg/cm²
Tadalafil dose: 3.57 mg /cm²
Tadalafil assay measured: 48.9% (w/w)

Content uniformity test has been evaluated, where tadalafil dose has been measured separately in each of 10 strips, as presented below. The obtained results confirmed the homogeneity of the film produced.

| **Measured dose of tadalafil/strip** | **% of declared dose** |
|---|---|
| **19.85 (max)** | 99.23% (max) |
| 19.63 | 98.13% |
| **19.57 (min)** | 97.87% (min) |
| 19.58 | 97.89% |
| 19.68 | 98.39% |
| 19.70 | 98.51% |
| 19.73 | 98.67% |
| 19.68 | 98.41% |
| **19.66 (average)** | **98.29% (average)** |

### Process parameters:

Equipment: laboratory scale Nanospider - needleless electrospinning
Electrode distance [mm] 140
Rotation/wire speed [mm] 15
Rewinding speed [mm/min] 5
EMW speed [mm /sec] 310
Air Input- flow [m3/h] 170.0
Air Output- flow [m3/h] 90
High Voltage setup
   HV CE Supply current [mA] 0.22
   HV CE Supply voltage [kV] -10.0
   HV SESupply current [mA] 0.22
   HV SE Supply voltage [kV] 50.0

## Claims

1. Suspension ready for the electro spinning, containing at least one hardly soluble active ingredient in the concentration of 1-30% and 2 to 30% of water soluble long-chained film forming polymers **characterized by** that no sedimentation is recorded within at least 6h, more preferably 8h, where sedimentation, evaluated based on assay difference between bottom and top layers of the 30-cm column of unmixed suspension, is no greater than 2.5%.

2. The suspension according to the claim 1, having a viscosity at most 500 mPas

3. The suspension according to 1 or 2 in which minimal 50% of the active ingredient is suspended.

4. The suspension according to 1; 2 or 3 in which the particle size of the undissolved active ingredient is **characterized by** D(90) not greater than 30 µm..

5. The suspension according to 4 in which 90% of all solid particle are greater than 500 nm and lower than 10µm.

6. The suspension according to the claims 1 to 5 in which the film forming polymer is selected from cellulose and its derivatives polyoxyethylene glycols and their derivatives and copolymers, polysaccharides, polyacrilic acid, its derivatives and copolymers, methacrylic acid derivatives, xanthan gum, alginates, carrageenan, pectins, Gantrez, maltodextrins or their mixtures.

7. The suspension according to the claim 6, in which the cellulose derivatives has been selected from methylcellulose, hydroxyethylcellulose, hydroxypropylcellulose, hydroxypropylmethylcellulose, sodium carmellose.

8. The suspension according to the claim 6 in which the polysaccharide is pullulan.

9. A method for preparing oral dispersible films of medical usable active ingredient with low solubility using the suspension according to the claims 1 to 7 by needleless electrospinning **characterized by** that the producing films' content deviation was at most ± 2.5%

10. The method according to the claim 9 **characterized by** parameters electrode distance 130 to 190 mm, rewinding speed 5 to 20 mm/ min; HV CE supply voltage -20 to -5 kV; HV SE supply voltage 30 to 70 kV; air humidity 0 to 50% RH.

11. The method according to the claim 10 **characterized by** parameters electrode distance 140 to 170 mm, rewinding speed 8 mm/ min; HV CE supply voltage -10 kV; HV SE supply voltage 50 kV; air humidity 10 to 25% RH.
